# EUROPEAN PATENT SPECIFICATION

(11) **EP 1 489 171 B1**
(45) Date of publication and mention of the grant of the patent: **31.10.2012**
(21) Application number: 03708622.0
(22) Date of filing: 14.03.2003
(51) Int. Cl.: C12N 15/11, C12N 15/10, A61K 31/7115

(54) **FUNCTIONAL MOLECULE AND PROCESS FOR PRODUCING THE SAME**
FUNKTIONELLES MOLEKÜL UND VERFAHREN ZUR HERSTELLUNG DAVON
MOLECULE FONCTIONNELLE ET SON PROCEDE DE PRODUCTION

(30) Priority: 19.03.2002 JP 2002077040
(43) Date of publication of application: 22.12.2004
(73) Proprietor: FUJITSU LIMITED, Kawasaki-shi, Kanagawa 211-8588 (JP)
(72) Inventor: FUJIHARA, Tsuyoshi, FUJITSU LIMITED, Kawasaki-shi, Kanagawa 211-8588 (JP); FUJITA, Shozo, FUJITSU LIMITED, Kawasaki-shi, Kanagawa 211-8588 (JP); TAKEISHI, Shunsaku, FUJITSU LIMITED, Kawasaki-shi, Kanagawa 211-8588 (JP)
(74) Representative: Fenlon, Christine Lesley
(86) International application number: PCT/JP2003/003087
(87) International publication number: WO 2003/078623

(56) References cited:
- EP-A1- 1 201 751
- WO-A-00/23456
- WO-A-00/24404
- WO-A-92/05285
- WO-A-92/14842
- WO-A1-92/03461
- WO-A1-96/40717
- WO-A2-99/21873
- US-A- 5 756 291
- KUSSER W: "CHEMICALLY MODIFIED NUCLEIC ACID APTAMERS FOR IN VITRO SELECTIONS: EVOLVING EVOLUTION" REVIEWS IN MOLECULAR BIOTECHNOLOGY, ELSEVIER, AMSTERDAM,, NL, vol. 74, no. 1, March 2000 (2000-03), pages 27-38, XP008042698 ISSN: 1389-0352
- SAKTHIVEL K ET AL: "Expanding the potential of DNA for binding and catalysis : highly functionalized dUTP derivatives that are substrates for thermostable DNA polymerases" ANGEWANDTE CHEMIE. INTERNATIONAL EDITION, WILEY VCH VERLAG, WEINHEIM, DE, vol. 37, no. 20, 2 November 1998 (1998-11-02), pages 2872-2875, XP002175620 ISSN: 1433-7851
- LATHAM J A ET AL: "The application of a modified nucleotide in aptamer selection : novel thrombin aptamers containing 5-(1-pentynyl)-2'-deoxyuridine" NUCLEIC ACIDS RESEARCH, OXFORD UNIVERSITY PRESS, SURREY, GB, vol. 22, no. 14, 1994, pages 2817-2822, XP002176163 ISSN: 0305-1048
- BRODY E N ET AL: "THE USE OF APTAMERS IN LARGE ARRAYS FOR MOLECULAR DIAGNOSTICS" MOLECULAR DIAGNOSIS, NAPERVILLE, IL, US, vol. 4, no. 4, December 1999 (1999-12), pages 381-388, XP009000095 ISSN: 1084-8592
- GILBERT B A ET AL: "RNA aptamers that specifically bind to a K Ras-derived farnesylated peptide." BIOORGANIC & MEDICINAL CHEMISTRY JUN 1997, vol. 5, no. 6, June 1997 (1997-06), pages 1115-1122, XP002473667 ISSN: 0968-0896
- JHAVERI S D ET AL: "In vitro selection of RNA aptamers to a protein target by filter immobilization." CURRENT PROTOCOLS IN MOLECULAR BIOLOGY / EDITED BY FREDERICK M. AUSUBEL ... [ET AL.] MAY 2001, vol. Chapter 24, May 2001 (2001-05), page Unit 24.3, XP002473668 ISSN: 1934-3647
- LIN Y. ET AL.: 'High-affinity and specific recognition of human thyroid stimulating hormone (hTSH) by in vitro-selected 2'-amino-modified RNA' NUCLEIC ACIDS RES. vol. 24, no. 17, 1996, pages 3407 - 3414, XP000625346

### SEQUENCE LISTING

<110> FUJITSU LIMITED
<120> FUNCTIONAL MOLECULE AND PROCESS FOR MANUFACTURING THE SAME
<130> 0241778
<140>
   <141>
<160> 1
<170> PatentIn Ver. 2.1
<210> 1
   <211> 14
   <212> DNA
   <213> Artificial Sequence
<220>
   <223> Description of Artificial Sequence:DNA for manufacturing a derivative which may bond to human albumin
<400> 1
   atgctctagc ccct 14

## Description

### Technical field

The present invention relates to a process for producing a functional molecule that shows high affinity for various targets (targeted materials), is easily amplified and replicated, is suitable for use in a wide range of fields such as drugs, drug delivery, biosensors, regulation of gene expression level, conquest of diseases caused by abnormal genes, analysis of functions of a protein translated by a gene, and development of reaction catalysts, and is particularly suitable for use in an analysis of proteins.

### Background art

In 2002, a working draft of human genome was published, and in 2003, the complete information thereof is going to be announced. Therefore, a main interest of researchers and scientists has been moving forward to an analysis of proteins which are gene products resulted from gene expression. In the analysis of proteins, it can be said that the analysis cannot be conducted without the presence of molecules having affinity for each targeted proteins. Various kinds of the proteins, which are the subjects for the analysis, are present in a cell and many of them have completely unknown amino acid sequences and structures thereof. Therefore, various molecules are required for the analysis of the proteins.

However, at present, there has been known no effective process for preparing or obtaining the molecules used for the analysis of proteins. The conventional and most common process for obtaining the molecule having affinity for a specific protein is a process to select an affinity antibody by utilizing an animal immune system. However, since an animal is used in this process, a large amount of the protein is required, and there are a large number of steps. Thus, the cost thereof becomes high. In addition, although the affinity antibody selected and obtained by this process can be replicated by making it as a monoclonal antibody followed by maintaining the cell, it requires a high cost and cannot be modified. Furthermore, there is a problem in that only an affinity antibody having affinity for a single target can be selected. Therefore, it is very difficult to select and to obtain affinity antibodies respectively having affinity for all of the proteins present in the cell.

Recently many studies have been conducted on a selection of a molecule having a specific function from a nucleotide random sequence, and a determination of the molecule on the basis of genetic information thereof. As the result,there have been many reports on molecules having affinity for a specific target and RNAs catalyzing a specific reaction.

However, a number of constitutional units of a nucleotide is four in both RNA and DNA, and thus kinds of functional groups present in a polymer thereof are very limited. Therefore, the function of such polymer is also limited. In order to solve this problem, a study has been conducted by using a modified nucleotide. However, it is necessary to remove a natural nucleotide corresponding to a modified nucleotide from an experimental system in order to determine that a molecule in which the nucleotide is selectively introduced at a certain region is the molecule having affinity for the target. As the result, the number of the constitutional units of the nucleotide is still the same, namely 4 + 1 - 1 = 4, and thus the fundamental problem has not yet been solved. Moreover, RNA is enzymatically very unstable. Therefore, synthesis of a molecule having the same function has been attempted by using a DNA that is enzymatically stable, but not succeeded yet. In these cases, it is necessary to repeat the operations of amplification and purification for obtaining a molecule having affinity for a specific target, hence it is still difficult to synthesize various molecules.

There have been studies that use amino acid or an artificial material as a constitutional unit of a molecule, and select the molecule in accordance with combinatorial chemistry. However, as the molecule itself does not have genetic information, the sequence information thereof cannot be amplified. Therefore, many steps are required for determining the sequence of the molecule.

Meanwhile, concerning the synthesis of the protein having the genetic information, there has been the study in which puromycin is introduced at a 3' terminal of mRNA (refer to a patent document 1). This study applies the property that puromycin is misunderstood as the amino acid in a translation system and is apt to easily be incorporated in the protein. However, in this case, an efficiency of puromycin incorporation is very low, and hence there has been, so far, a report only describing that the molecule is selected from a library of random 3 residues of amino acids. Moreover, there is the problem in that, even if the efficiency of puromycin incorporation is improved, a number of a library pool that possibly becomes the protein having the genetic information is very small within the DNA random pool. Such problem arises from the fact that a termination codon is present in a DNA random sequence in a proportion of 3/64 and a rare codon which is not preferred by the cell is present.
Patent document 1:
   Japanese Patent Application Laid-Open (JP-A) No. 2002-291491

The present invention aims at providing a functional molecule, which has such affinity and specificity for a target that are comparable to the affinity and specificity of an antibody against an antigen, is capable of functioning as the antibody sensu stricto,is capable of recognizing a wide range of targets, shows high affinity for various kinds of targets (targeted materials, detection targets), is easy to be amplified and replicated, is preferably applicable in a wide range of fields such as drugs, drug delivery, biosensors, regulation of gene expression level, conquest of diseases caused by an abnormal gene, elucidating the function of the protein translated from a gene and development of reaction catalysts, and is particularly preferable for an analysis of a protein.

In addition to the above, the present invention also aims at providing a process for producing a functional molecule, which does not require repetition of complicated operations of amplification and purification of the functional molecule, and realizes effective and collective production of the functional molecule(s).

### Disclosure of the Invention

The present invention is defined in and by the appended claims.

A process for producing a functional molecule according to the present invention includes a producing step which is a step of randomly polymerizing modified nucleotide N-mer so as to produce modified oligonucleotide sequences, where N represents a numerical term, wherein each of the modified nucleotide N-mer contains a modified nucleoside prepared by introducing a substituent into a nucleoside composing a nucleic acid. According to the process for producing a functional molecule of the present invention, in the producing step, the modified oligonucleotide sequences are produced by randomly polymerizing modified nucleotide N-mer, where n represents a numerical term, containing a modified nucleoside prepared by inserting a substituent into a nucleoside composing a nucleic acid. The modified oligonucleotide sequence has more substituents which recognize a target than 4 kinds of nucleotides that compose a nucleic acid, maintains a self-replicating ability of a nucleic acid as it is, and has affinity and specificity comparable to those of an antibody. Therefore, by amplifying and replicating the modified oligonucleotide sequence, various types of the functional molecules that recognize the target can be efficiently produced.

The functional molecule according to the present invention is produced by the process for producing a functional molecule of the present invention has affinity and specificity for the target, that are comparable to the affinity and specificity for an antibody against an antigen, can recognize various types of a target, and is suitable for use in a wide range of fields such as drugs, drug delivery, biosensors, regulation of gene expression level, conquest of diseases caused by gene abnormality, analysis of the function of the protein translated by the gene, and developing reaction catalysts.

### Brief Description of the Drawings

FIG. 1 is a diagrammatic view showing an example of an oligonucleotide comprised of a modified oligonucleotide and a fixed oligonucleotide, which composes a random polymer pool.
FIG. 2 is a schematic view showing an example of a DNA synthesizer used for synthesizing the random polymer pool.
FIG. 3A is a schematic view explaining a state where the random polymer pool is synthesized in the process for producing a functional molecule according to the present invention.
FIG. 3B is a schematic view showing an example of an affinity column.
FIG. 3C is a schematic view explaining a state where the oligonucleotide containing the modified oligonucleotide random sequence is selected and collected.
FIG. 3D is a schematic view explaining a state where the selected modified oligonucleotide sequence is amplified by PCR, and the sequence thereof is determined and translated.

### Best Mode for Carrying Out the Invention

### (Functional molecule and process for producing the same)

A functional molecule is produced by the process for producing a functional molecule according to the present invention. Below, details of the functional molecule produced by the process according to the present invention will be described along with the explanations of the process for producing a functional molecule of the present invention.

The process for producing a functional molecule according to the present invention includes at least a producing step, preferably further includes a selecting step, a determining step, and a translating step, and optionally further includes other steps appropriately selected depending on the purpose.

### Producing step

The producing step is a step of randomly polymerizing modified nucleotide N-mer so as to produce modified oligonucleotide sequences, where n representing a numerical term, wherein each of the modified nucleotide N-mer contains a modified nucleoside prepared by introducing a substituent into a nucleoside composing a nucleic acid.

A reaction product, which contains the modified oligonucleotide sequences obtained as a result of the producing step, can be used in the selecting step and the like as a random polymer pool containing the modified oligonucleotide sequences without separation and purification processing.

The modified nucleotide N-mer comprises the modified nucleoside.

The modified nucleoside is prepared by introducing a substituent into a nucleoside composing the nucleic acid.

"N" in the modified nucleotide N-mer represents a numerical term, is preferably di- or larger, is more preferably selected from di- to deca-, and is particularly preferably di- or tri-.

When the N is smaller than di-, a number of varieties of the modified nucleotide is almost the same as that of nucleotide composing a nucleic acid, i.e., four. Therefore, the functional molecule resulted from such the modified nucleotide may not have the improved recognition ability for a target. When the N is tetra- or larger, one base deletion or one base addition, which may be caused during the amplification step, cannot be easily distinguished from others due to the similarity in the base sequences. When N is tri-, sixty-four different substituents can be introduced in maximum, and thus various kinds of proteins will be formed with twenty kinds of amino acids. Therefore, tri- is satisfactory for N. Compared to the case where N is tri-, the modified nucleotide in which N of tetra- or larger numerical terms does not provide sufficient expected effects, and moreover there is concern that a load on the synthesis may be increased.

The nucleic acid includes either DNA or RNA, and the DNA or RNA may have a single-stranded structure or a double-strand structure.

The DNA has four kinds of bases, which are adenine (A), thymine (T), guanine (G) and cytosine (C), and the bases in DNA polynucleotide chains are present so as to projecting towards inside in a plane vertical to the central axis of the DNA polynucleotide chains, r namely making the so-called Watson-Crick base pairs. Here, thymine and cytosine specifically bind to adenine and guanine, respectively, by hydrogen bonding. As the result, in the double-stranded DNA, two polypeptide chains are complementarily bound to each other.

The four kinds of nucleosides (deoxyribonucleosides) of the DNA are deoxyadenosine (dA), deoxyguanosine (dG), deoxycytidine (dC) and deoxythymidine (dT).

The RNA has four kinds of bases, which are adenine (A), thymine (T), uracil (U), and cytosine (C), and can be classified into three types, which are ribosome RNA (rRNA), transfer RNA (tRNA) and messenger RNA (mRNA).

The four kinds of nucleosides (ribonucleosides) of the RNA are adenosine (A), guanosine (G), cytidine (C), and uridine (U).

When N is di- in the modified nucleotide N-mer, the modified nucleotide dimer is not restricted, and can be appropriately selected depending on the purpose. For example, it may be made of a combination of four kinds of nucleosides composing a nucleic acid and the modified nucleoside, or may be made of a combination of the modified nucleosides. In this connection, the substituent may not be introduced into the nucleoside (a sugar is bound to a purine or pyrimidine base), but instead, may be introduced into nucleotide (a phosphate group is added to the nucleoside).

A position of the substituent to be introduced in the modified nucleoside is not restricted, and can be appropriately selected depending on a purpose. Examples of the position includes the 5th position in pyrimidine, the 7th position in purine, the 8th position in purine, substitution of an exocyclic amine, substitution of 4-thiouridine, substitution of 5-bromo, substitution of 5-iodide uracil, and the like. Among them, the 5th position in pyrimidine and the 7th position in purine are preferable as an enzyme reaction is scarcely inhibited at amplification (replication), and the 5th position in pyrimidine is more preferable because of easiness of synthesis.

A process for introducing a substituent into the nucleoside is not restricted, and can be appropriately selected depending on the purpose. For example, the process is preferably the process shown in the following formula, in which substituent R is introduced at the 5th position of pyrimidine base in the nucleoside.

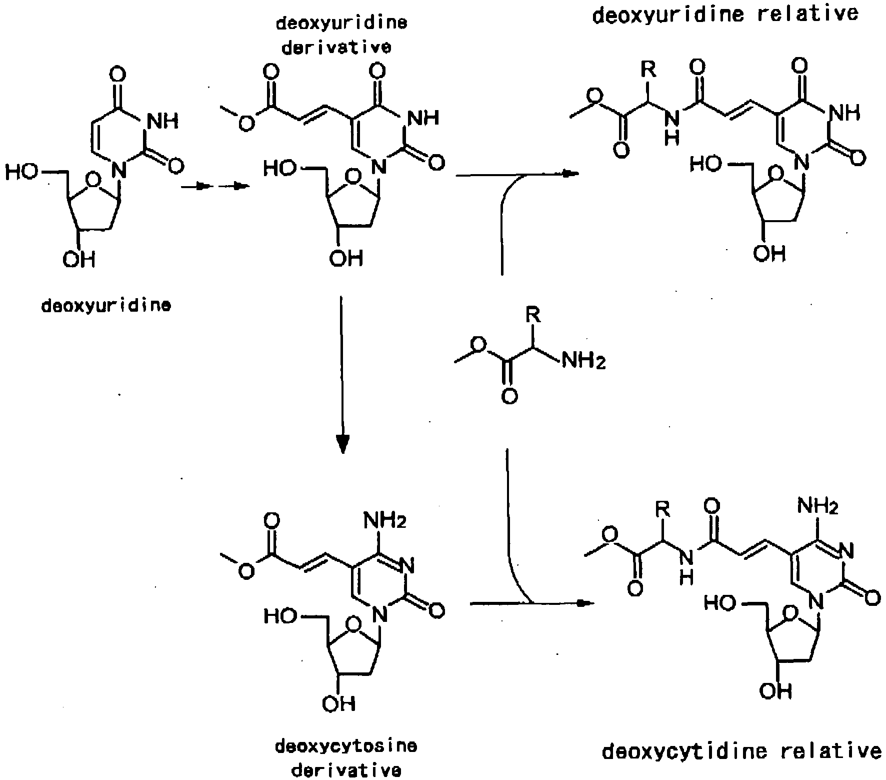

The substituent R is not restricted, and can be appropriately selected depending on the purpose. For example, the substituent R includes natural or unnatural amino acids, metal complexes, fluorescent dyes, oxidation-reduction coloring agents, spin label compounds, hydrogen atom, C1-10 alkyl groups, and groups represented by the following formulae (1) to (10).

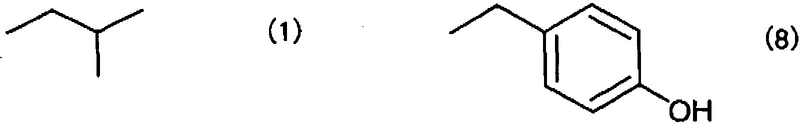

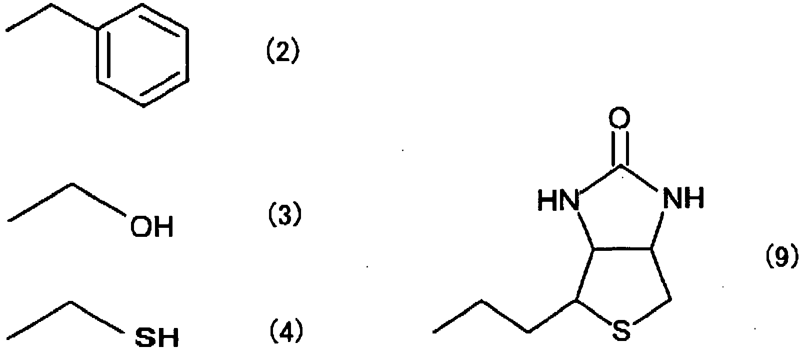

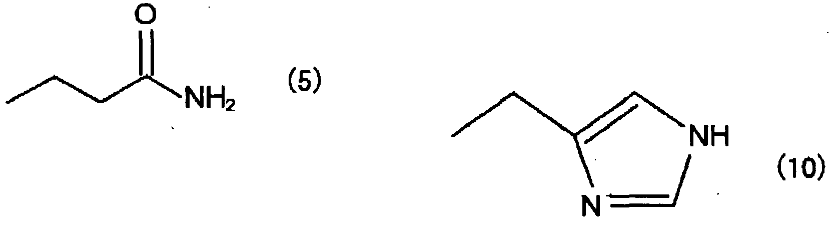

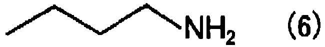

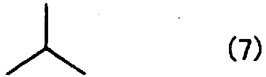

The natural or unnatural amino acids are not restricted, and can be appropriately selected depending on a purpose. Examples of the natural or unnatural amino acids include valine, leucine, isoleucine, alanine, arginine, glutamine, lysine, aspartic acid, glutamic acid, proline, cysteine, threonine, methionine, histidine, phenylalanine, tyrosine, tryptophane, asparagine, glycine, serine, and the like.

The metal complexes are not restricted, and can be appropriately selected depending on the purpose as long as they are compounds in which a ligand is coordinated with a metal ion. Examples thereof include Ru-bipyridil complexes, ferrocene complexes, nickel imidazole complexes, and the like.

The fluorescent dyes are not restricted, and can be appropriately selected depending on the purpose. Examples thereof include fluorescent dyes such as fluorescein dyes, rhodamine dyes, eosine dyes, NBD dyes, and the like.

The oxidation-reduction coloring agents are not restricted, but can be appropriately selected depending on the purpose. Examples thereof include leuco dyes such as leucoaniline, leucoanthocyanine, and the like.

The spin label compounds are not restricted, and can be appropriately selected depending on the purpose. Examples thereof include iron N-(dithiocarboxy) sarcosine, TEMPO (tetramethyl piperidine) derivatives, and the like.

The C1-10 alkyl groups are not restricted, and can be appropriately selected depending on the purpose. Examples thereof include a methyl group, ethyl group, propyl group, isopropyl group, butyl group, isobutyl group, tert-butyl group, pentyl group, neopentyl group, hexyl group, cyclohexyl group, octyl group, nonyl group, decyl group and the like.

These may be further substituted with a substituent.

The process for synthesizing the modified nucleotide dimer is not restricted, and can be appropriately selected depending on the purpose. Examples thereof include a diester method, triester method, phosphite method, phosphoramidite method, H-phosphonate method, thiophosphite method, and the like. Among these methods, the phosphoramidite method is preferable.

In the phosphoramidite method, a condensation reaction of nucleoside phosphoramidite and the nucleoside is generally carried out as a key reaction by using tetrazole as an enhancer. This reaction usually occurs competitively in both a hydroxyl group of a sugar part and an amino group of a base part of the nucleoside. However, in order to realize the desired nucleotide synthesis, the reaction is selectively induced only in the hydroxyl group of the saccharide part. Hence, for inhibiting a side reaction to the amino group, it is modified with a protecting group. For example, the modified nucleotide dimer (AU₁) can be, as shown by the following formula, synthesized from deoxyadenosine and modified deoxyuridine.

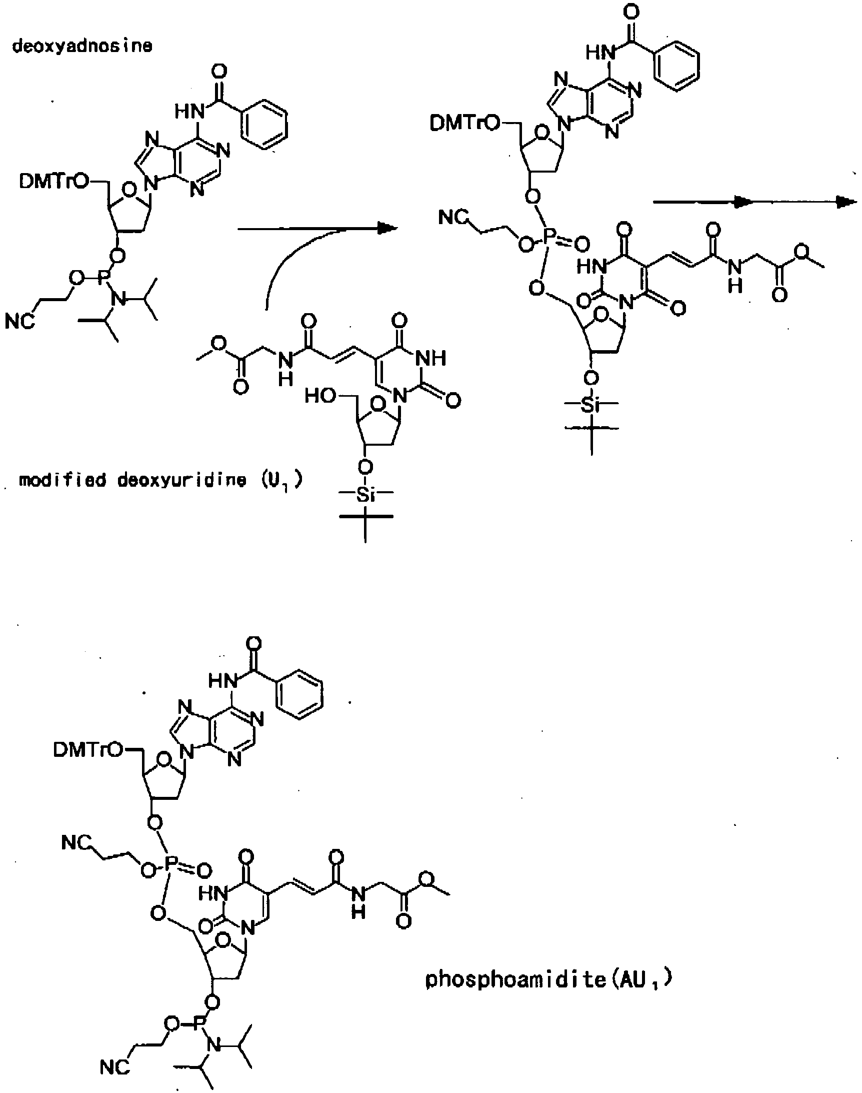

In the above formula, DMTr represents a dimethoxy trityl group.

The modified nucleotide dimers (AC₁, C₂A, C₃C, C₄G, C₅T, GC₆, GU₂, U₃A, U₄C, U₅G, and U₆T), which are represented in the relation table of the following Table 1 mentioned later, can be also synthesized by the same method.

Here, each one of the synthesized modified nucleotide dimers is made connected (related) with each one of sixteen nucleosides that are present as one-to-one combinations of four kinds of a nucleoside in the relation table.

When a number of varieties of the modified nucleotide dimers is less than five, there is no large difference from that of four kinds of nucleotides composing the nucleic acid, and thus the recognition ability for a target may not be sufficiently improved.

The relation table is, for example, exemplified as shown in Table 1 below. In Table 1, four bases of a nucleoside are transversely (5' direction) arranged in the order of A, C, G and T, are longitudinally (3' direction) arranged in the order of A, C, G and T, and sixteen patterns (boxes) are formed by relating these bases one-by-one.

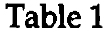

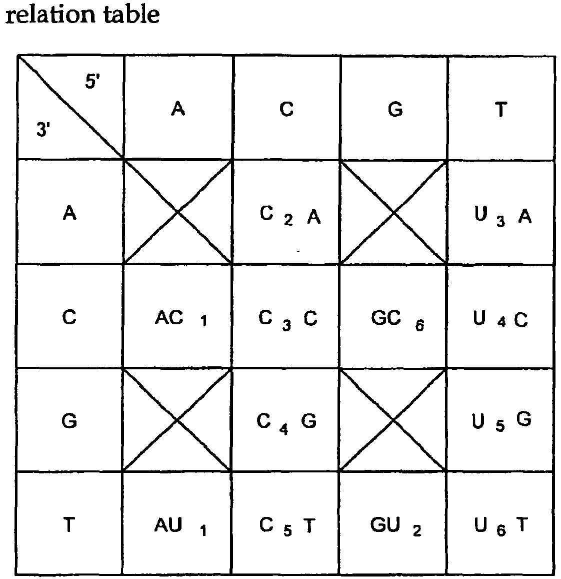

A modified nucleotide trimer can be used in the similar manner to that of the modified nucleotide dimer. Examples of the relation table in this case include Table 2 shown below. In Table 2, fifty-six patterns (fifty-six kinds of modified nucleotide trimers) are formed. Similarly to the case of the modified nucleotide dimer and the modified nucleotide trimer, a modified nucleotide N-mer (n representing the numerical term) can be used. In the relation table in this case, 4^{N} patterns (4^{N} modified nucleotide N-mer) can be made. Here, "N" of 4^{N} represents an integer corresponding to the numerical term N of N-mer.

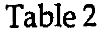

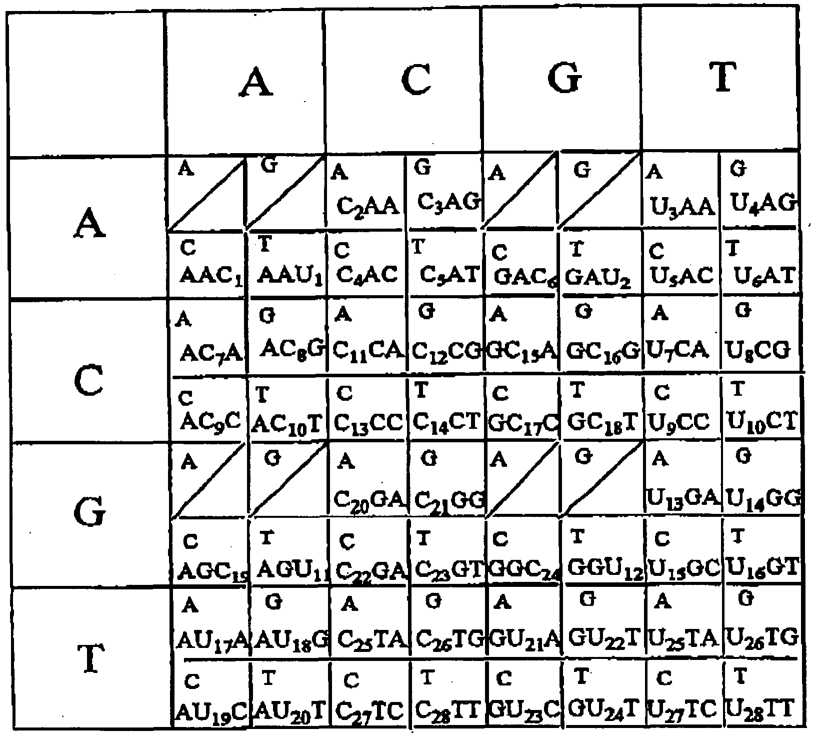

In the relation table in Table 1, twelve modified nucleotide dimers are conditioned as below.

Specifically, a base sequence is read in the 5' - to - 3' chain direction, and a base sequence AC corresponds to the modified nucleotide dimer AC₁. A sequence AT corresponds to the modified nucleotide dimer AU₁. A base sequence CA corresponds to the modified nucleotide dimer C₂A. A base sequence CC corresponds to the modified nucleotide dimer C₃C. A base sequence CG corresponds to the modified nucleotide dimer C₄G. A base sequence CT corresponds to the modified nucleotide dimer C₂A. A base sequence GC corresponds to the modified nucleotide dimer GC₆. A base sequence GT corresponds to the modified nucleotide dimer GU₂. A base sequence TA corresponds to the modified nucleotide dimer U₃A. A base sequence TC corresponds to the modified nucleotide dimer U₄C. A base sequence TG corresponds to the modified nucleotide dimer U₅G. A base sequence TT corresponds to the modified nucleotide dimer U₆T.

Conditioning of a base sequence and the modified nucleotide dimer in the relation table of Table 1 is not restricted, and can be appropriately selected depending on a purpose. It should be noted that Table 1 is simply an example. When it is difficult to prepare twelve modified nucleotide dimers, some of the base sequences may be conditioned with the identical modified nucleotide dimers. However, it may result in lowering the recognition ability for the target.

For AA, AG, GA and GG that are combinations of purine bases in the relation table of Table 1, no modified nucleotide dimer is prepared due to a lower reactivity of an enzyme used for modifying the purine base. However, this does not mean that the modified nucleotide dimer containing the purine bases cannot be prepared.

By making a relationship between twelve modified nucleotide dimers and nucleotides on the basis of the relation table of Table 1, variations of the base sequence are increased to from four kinds in the conventional nucleic acid to twelve kinds, and thus the resulted molecule therefrom is able to exercise recognition ability for many kinds of a target.

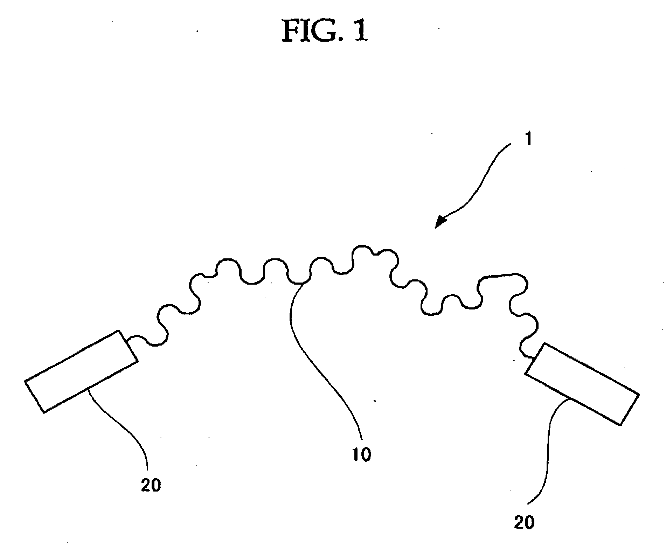

The oligonucleotide 1 composing the random polymer pool preferably has fixed oligonucleotide sequences 20 at both ends of the modified oligonucleotide sequence 10 as shown in FIG. 1 in view of that an amplification (replication) can be efficiently carried out by binding a primer to a region of the fixed oligonucleotide sequence in the PCR method, during the below-described determining step.

The number of nucleotides contained in the modified oligonucleotide sequence is not restricted, and can be appropriately selected depending on the purpose. It is preferably from 10 to 100, and more preferably 10 to 50.

The number of nucleotides contained in the fixed oligonucleotide sequence is not restricted, and can be appropriately selected depending on the purpose. It is preferably 15 or more, and more preferably 20 to 40.

The process for producing the random polymer pool is not restricted, and can be appropriately selected from well known methods in the art depending on the purpose. Examples thereof include a method using a DNA synthesizer (automated DNA synthesizer), and the like.

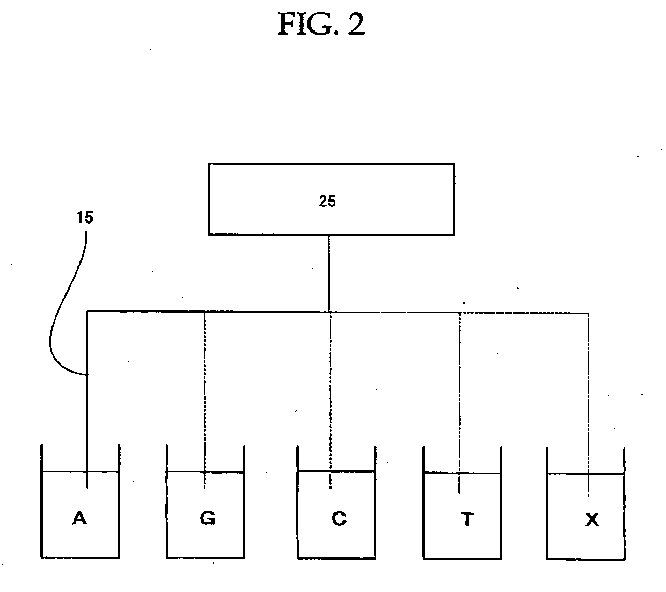

The method using the DNA synthesizer (automated DNA synthesizer) is not restricted, and can be appropriately selected depending on the purpose. The preferable method thereof is a process which employs the DNA synthesizer (automated DNA synthesizer) as shown in FIG. 2, uses a mixture (there are twelve kinds in the example shown in FIG. 2; expressed as "X" in FIG. 2) of the synthesized modified nucleotide dimers as a reagent, and sucks this reagent up by a nozzle 15 so as to polymerize under the control by means of a controller 25 to thereby prepare the random polymer pool having the modified oligonucleotide sequences with various random sequence arrangements. This method is advantageous as the random polymer pool is efficiently prepared.

For synthesizing the fixed nucleotide sequence, a similar method to the above can be used by using four kinds of base, i.e., adenine (A), thymine (T), guanine (G) and cytosine (C.)

The process for producing the random polymer pool also includes a process in which monomer blocks are aligned to the previously prepared oligonucleotide random sequence, annealing is carried out thereon, and the monomer blocks are joined each other by a DNA ligase or an RNA ligase, to thereby prepare a random polymer pool. Also in this case, the process can prepare the random polymer pool containing the modified oligonucleotide sequences with various random sequence arrangements.

The DNA ligase is an enzyme to catalyze formation of a covalent bond between the 5' phosphate group and the 3' hydroxyl group of adjacent nucleotides.

The RNA ligase is an enzyme to join a polynucleotide having the terminal 5' phosphate group to a polynucleotide having the terminal 3' hydroxyl group. A substrate of the RNA ligase is normally RNA, and the RNA ligase efficiently joins a polydeoxyribonucleotide having the terminal of 5' phosphate group to a polydeoxyribonucleotide having ribonucleotide only at 3' terminal.

The random polymer pool contains the modified oligonucleotide sequences and, and if necessary, may further contain a sequence which is prepared by randomly polymerizing unmodified monomers or oligomers of the DNA or RNA.

### Selecting step

The selecting step is a step of selecting the modified oligonucleotide sequence having affinity for the target from the prepared modified oligonucleotide sequences.

The method of the selection is not restricted, and can be properly selected from well known methods in the art depending on the purpose. Examples thereof include various methods such as affinity chromatography, filter binding assay, liquid-liquid partition, filtration, gel shift, and density gradient centrifugation. One of these methods may be conducted independently, or two or more of these methods may be conducted in combination in the selection step. Among these methods, the affinity chromatography is preferable.

The affinity chromatography is a method of a separation and purification utilizing biological affinity by which specific components are easily bound to each other. Specifically, the target is immobilized on column filler such as a resin, and is equilibrated with a buffer solution for attachment. Thereafter, a solution containing the random polymer pool is poured into the column to leave stand under a certain condition. The modified oligonucleotide sequence having affinity for the target is adsorbed onto the column, and sufficient washing is carried out with the buffer solution for attachment so as to remove the components other than the adsorbed modified oligonucleotide sequence. By passing a solution containing the target or a diluted buffer solution through the column, the oligonucleotide containing the modified oligonucleotide sequence can be collected, and selected.

In the case where the target is unknown and a wide variety thereof is present (for example, organs and blood serum), a method of the selection is preferably a method in which two or more targets are immobilized on a matrix having an one to three dimensional spatial arrangement, the random polymer pool containing the modified oligonucleotide sequence is made to interact with the matrix on which the target has been immobilized, and then the modified oligonucleotide sequence attached to the target is purified.

The process for immobilizing the target on the matrix is not restricted, and can be appropriately selected depending on the purpose. When the target is a protein, examples of such the process include: a western blotting method in which the target is subjected to polyacrylamide electrophoresis (for example, SDS-PAGE and the like), and then is transferred to the membranous matrix; a dot blotting method or a slot blotting method in which the target or diluted solution thereof is directly impregnated into the membranous matrix; and the like. Among these methods, the western blotting method is preferable as a trace amount of the protein contained in the solution of a complicated composition such as a cell extract can be clearly detected.

The western blotting method is an approach for detecting a specific protein from a protein mixture by using a combination of excellent separation performance of electrophoresis and high specificities of an antigen-antibody reaction, and is also a method in which the protein is electrically moved and immobilized from a gel to the aforementioned membranous matrix after conducting SDS-PAGE, isoelectric focusing, 2-dimensional electrophoresis, or the like. The membranous matrix is not restricted, and can be appropriately selected depending on the purpose. Examples thereof include highly hydrophobic nitrocellulose film to which the protein is easily bound, and a PVDF (polyvinylidene difluoride) film which has excellent hydrophobicity.

In the present invention, it is preferred that the target is labeled with at least one labeling material selected from radioisotopes, chemiluminescence materials, fluorescent materials, enzymes, antibodies, ligands and receptors; or the modified oligonucleotide sequence contained in the random polymer pool is labeled with at least one labeling material selected from radioisotopes, chemiluminescence materials, fluorescent materials, enzymes, antibodies, ligands and receptors.

Examples of the radioisotope include ³²P, ³³P ³⁵S and the like.

Examples of the chemiluminescence material include acridinium ester, luminol, isoluminol, or these derivatives, and the like. Detection by using the chemiluminescence requires exposure to an X-ray film or a scanner capable of detecting chemiluminescence However, the detection by the chemiluminesence has such an advantage that a trace of protein can be easily detected, as the chemiluminesence has a higher sensitivity than other coloration methods by 10 to 50 folds or more.

Examples of the fluorescent material include fluorescent dyes such as fluorescein series, fluorescein series, rhodamine series, eosine series and NDB series, green fluorescent protein (GFP), chelates of rare earths such as europium (Eu), terbium (Tb) and samarium (Sm), tetramethyl rhodamine, Texas Red, 4-methyl umbelliferone, 7-amino-4-methyl coumarin, Cy3, Cy5, and the like.

Examples of the enzyme include peroxidase, alkaline phosphatase, glucose oxidase, beta-galactosidase, luciferase, and the like.

In addition to the ones listed above, examples of the labeling materials include biotin, ligand, a specific nucleic acid, protein, hapten, and the like. Possible combinations of the labeling material and the target or modified oligonucleotide sequence to be labeled are: biotin as the labeling material and avidin or streptavidin specifically binding thereto; hapten as the labeling material and an antibody specifically binding thereto; a ligand as the labeling material and a receptor; and a specific nucleic acid, protein, hapten or the like as the labeling material and a nucleic acid specifically binding thereto, a nucleic acid-binding protein, or a protein having the affinity for the specific protein; and the like.

The process for purification is not restricted, and can be appropriately selected depending on the purpose. For example, a particularly preferable method is a method in which the modified oligonucleotide sequence, which has been immobilized on a predetermined position (region) of the membranous matrix (a membrane such as a nitrocellulose film) by the western blotting method and attached to the target, is cut at every predetermined position (region) together with the matrix (the membrane such as a nitrocellulose film) to thereby yield a cut piece, and then the modified oligonucleotide sequence is collected from the cut piece. This method is advantageous as no special purification process is required and the modified oligonucleotide sequence can be efficiently purified in a simple manner. Also, it is advantageous in view of that the different modified oligonucleotide sequences depending on the predetermined positions (regions) can be efficiently purified.

The collecting of the modified oligonucleotide sequence from the cut piece can be carried out by applying a voltage and using an ionic solution in accordance with electroelution, or by using a hot water.

The purification may be carried out by an another method, in which the modified oligonucleotide is dissociated from the target, while monitoring dissociation constants of the target and the modified oligonucleotide sequence. In this case, the functional molecule having the desired dissociation constant can be selected through one processing in minimum. By measuring the dissociation constants and controlling the dissociation constants in this manner in the selecting step, the functional molecule can be efficiently selected. Note that, the dissociation constant can be appropriately set depending on the target. For example, it can be measured by using a measuring instrument using warbis plasmon resonance.

In selection step of the present invention, by using an interaction between two or more molecules having mutually different dissociation constants, for example, the functional molecule is selected by a washing operation suitable for a small dissociation constant on one hand, and a carrier for the selection can be regenerated by a drastic washing operation suitable for a large dissociation constant on the other hand. In this method, even when there are two or more targets, a plurality of the functional molecules can be efficiently selected at once by using a single substrate, as the regeneration is possible. Therefore, it is possible to eliminate waste losses and to reduce the cost.

In the selecting step, the functional molecule can also be selected by a method that, by corresponding each of a plurality of molecules to each of linkers having different reactivities, the functional molecules recognizing each molecules can be sequentially selected by different post treatments. In this way, a plurality of the functional molecules can be efficiently selected using a single random polymer pool.

Examples of the different post treatments include a process for flowing a reagent capable of adjusting a binding strength of the linker, a process for flowing a reagent capable of adjusting a cleaving easiness of the linker bond, and a process which utilizes a reaction that selectively cuts a specific region by an optical reaction or electrode reaction.

The target (targeted material) is not restricted, and can be properly selected in accordance with the purpose. Examples thereof include proteins, lipoproteins, sugar proteins, polypeptides, lipids, polysaccharides, lipopolysaccharides, nucleic acids, environmental hormones, drugs, a complex of these substances, and the like. These substances can be used independently and may be used in combination of two or more. Among them, preferable ones are plasma protein, tumor marker, apoprotein, virus, autoantibody, coagulation and fibrinolysis factor, hormone, drug in blood, HLA antigen, environmental hormone, nucleic acid, and the like.

Examples of the plasma protein include immunoglobulin (IgG, IgA, IgM, IgD, IgE), component of complement (C3, C4, C5, C1q), CRP, α α₁-antitrypsin, α₁-microglobulin, β₂-microglobulin, haptoglobin, transferrin, ceruloplasmin, ferritin, and the like.

Examples of the tumor marker include α-fetoprotein (AFP), carcinoembryonic antigen (CEA), CA19-9, CA125, CA15-3, SCC antigen, prostatic acid phosphatase (PAP), PIVKA-II, γ-seminoprotein, TPA, elastase I, nerve specific enolase (NSE), immunosuppressive acidic protein (IAP), and the like.

Examples of the apoprotein include apoA-I, apoA-II, apoB, apoC-II, apoC-III, apoE, and the like.

Examples of the virus include hepatitis type B virus (HBV), hepatitis type C virus (HBC), HTLV-I, HIV, and the like. In addition, infectious diseases caused by pathogens other than virus include ASO, toxoplasma, mycoplasma, STD, and the like.

Examples of the autoantibody include antimicrosome antibody, anti-thyroglobulin antibody, antinuclear antibody, rheumatism factor, antimitochondrial antibody, myelin antibody, and the like.

Examples of the coagulation and fibrinolysis factor include fibrinogen, fibrin degradation product (FDP), plasminogen, α₂-plasmin inhibitor, antithrombin III, β-thromboglobulin, factor VIII, protein C, protein S, and the like.

Examples of the hormone include pituitary hormones (LH, FSH, GH, ACTH, TSH, prolactin), thyroid hormones (T₃, T₄, thyroglobulin), calcitonin, parathyroid hormone (PTH), adrenocortical hormones (aldosterone, cortisol), gonadal hormones (hCG, estrogen, testosteron, hPL), pancreatic digestive tract hormones (insulin, C-peptide, glucagon, gastrin), others (renin, angiotensin I, II, enkephalin, erythropoietin), and the like.

The environmental hormone is exogenous endocrine disruptors widely distributed in the ambient world. The environmental hormone is taken in organisms in accordance with daily activities, and influences various physiological endocrine phenomena of the organisms, such as reproduction, ontogenesis, and behaviors. Examples of the environmental hormone include nonyl phenol, octyl phenol, bisphenol A, butyl benzyl phthalate, tributyltine, PCB, polychlorinated dibenzodioxin, polychlorinated dibenzofuran, dioxins, DDT, DDE, DDD, endosulfan, methoxychlor, heptachlor, toxaphen, dieldrin, lindane, diethylstilbestrol (DES), ethynyl estradiol (component of the Pill), coumestrol, formononetein, genistein, and the like.

Examples of the drug in blood include: antiepileptic agents such as carbamazepin, primidone and valproic acid; circulatory disease drugs such as digoxin, quinidine, digitoxin and theophylline; antibiotics such as gentamycin, kanamycin and streptomycin, and the like.

Examples of the nucleic acid include cancer-associated gene, gene associated with genetic disease, virus gene, bacterial gene, gene showing polymorphism called a risk factor of a disease, and the like.

Examples of the cancer-associated gene include k-ras gene, N-ras gene, p53 gene, BRCA1 gene, BRCA 2 gene, src gene, ros gene or APC gene, and the like.

Examples of the gene associated with genetic disease include those related to various inborn errors of metabolism such as phenylketonuria, alcaptonuria, cystinuria, Huntington's chorea, Down's syndrome, Duchenne muscular dystrophy, angiostaxis, and the like.

Examples of the virus and bacterial genes include hepatitis C virus, hepatitis B virus, influenza virus, measles virus, HIV, mycoplasma, Rickettsia, streptococcus, Salmonella, and the like.

Examples of the gene showing polymorphism include gene having the base sequence, which is not necessarily related directly to a cause of a disease and is different between individuals, such as PS1 (preselinin 1) gene, PS2 (preselinin 2) gene, APP (β-amyloid precursor protein) gene, lipoprotein gene, and gene associated with HLA (Human Leukocyte Antigen) or blood groups, or gene associated with onset of hypertension and diabetes.

Examples of a sample containing the target include: pathogens such as bacteria and virus, blood, saliva and an affected tissue piece collected from organisms; and excretes such as feces and urine. In the case for prenatal diagnosis, it may include a fetal cell present in amniotic fluid, a portion of dividing egg cells in vitro, and the like. These samples may be previously subjected to a cytocidal treatment such as an oxygen treatment, heat treatment, surfactant treatment, ultrasonication treatment or a combination of these operations directly, or as required, after being subjected to condensation by centrifugal operation or the like as sedimentation.

### The determining steps

The determining steps are steps for amplifying the selected modified oligonucleotide sequence in the selecting step, and determining the base sequence thereof.

The method of amplification is not restricted as long as it can amplify the number of the targeted modified oligonucleotide sequence, and can be appropriately selected from PCR (Polymerase Chain Reaction) and RT-PCR

The PCR stands for Polymerase Chain Reaction, and this method enables to amplify the specific oligonucleotide region by some hundred thousand folds by repeating the DNA synthesis reaction using DNA synthetase in vitro. According to PCR, the primer elongation reaction is carried out by taking four or five kinds of nucleotide triphosphate (deoxyadenosine triphosphate, deoxyguanosine triphosphate, deoxycytidine triphosphate, and deoxythymidine triphosphate or deoxyuridine triphosphate (a mixture of these compounds may be also called dNTP)) as a substrate into the primer.

For this elongation reaction, an amplification reaction reagent containing the unit nucleic acid and a nucleic acid elongation enzyme is usually used so as to amplifying a nucleic acid chain. In this case, the applicable nucleic acid elongation enzymes are arbitrary DNA polymerases such as E. coli DNA polymerase I, Klenow fragment of E. coli DNA polymerase I, T4 DNA polymerase and the like. Particularly, thermostable DNA polymerases such as Taq DNA polymerase, Tth DNA polymerase, Vent DNA polymerase and the like are preferably used. By using these enzymes, it is not necessary to add a new enzyme for every cycle, it becomes possible to automatically repeat the cycle, and an annealing temperature can be set at 50°C to 60°C. Therefore, the primer has the increased specificity of target base sequence recognition, and the gene amplification reaction can be quickly and specifically carried out (Japanese Patent Application Laid-Open (JP-A) No. 01-314965 and 01-252300).

In course of this reaction, oil can be added to prevent evaporation of water contained in a reaction solution. In this case, the oil is the one which is distributable from water, and has the smaller specific gravity than that of water. Specifically, examples thereof include silicone oil, mineral oil, and the like. Moreover, some gene amplification instruments do not require such the medium, and the primer elongation reaction can also be conducted by using such the gene amplification instruments.

As described above, repetitions of the elongation reaction by using the primer enable an efficient operation of gene amplification of the target oligonucleotide, and enable to yield a large amount of the oligonucleotide. The specific method including conditions for the gene amplification reaction is any of the well known methods described in references such as Zikken Igaku (Yodosha, 8, No. 9. 1990), PCR Technology (Stockton Press, 1989), and the like.

As a result of PCR, the modified oligonucleotide sequence is substituted with a natural oligonucleotide sequence having no modified substituent.

When the modified oligonucleotide sequence is not DNA but RNA, DNA can be synthesized by performing a reverse transcription reaction. The reverse transcription reaction is a process for synthesizing DNA using RNA as a template. A reaction solution and a reaction condition of the reverse transcription reaction are different depending on a target RNA. For instance, operations include adding an RNase-free sterilized distilled water and a 3'-primer to an RNA solution, incubating, and then cooling and adding a reverse transcription buffer solution, which includes Tris-HCl, KCl, MgCl₂ and the like, DTT, and dNTPs, and adding a reverse transcriptase followed by incubation. The termination of the reverse transcription reaction can be carried out by adjusting the conditions of the incubation. Such the reverse transcription reaction can also be performed by a reverse transcription PCR.

The method of determining the base sequence of the amplified oligonucleotide is not restricted, and can be appropriately selected from well known methods in the art depending on the purpose. Examples thereof include a method by using gene cloning, a chain terminator method, a Sanger method, a DNA sequencer (automated DNA base sequence determination equipment) by using a dideoxy method, and the like. One of these methods may be employed independently, or 2 or more methods may be employed in combination.

In the aforementioned gene cloning, the amplified oligonucleotide can be produced by transforming a host cell by using an expression vector, into which the amplified oligonucleotide sequence is incorporated, followed by culturing this transformant.

Examples of the expression vector include a plasmid vector, a phage vector, a chimera vector prepared from a plasmid and a phage, and the like.

Examples of the host cell include: prokaryotes such as Escherichia coli and Bacillus subtilis; eukaryotes such as yeast; animal cells; and the like.

### Translating step

The translating step is a step of translating the modified oligonucleotide sequence, of which base sequence has been determined, on the basis of a relation table, wherein the relation table shows relationships between at least 4^{N} nucleotide N-mer and at least the modified nucleotide N-mer, and wherein the 4^{N} nucleotide N-mer are presented as one-to-one combinations of four kinds of a nucleoside in the relation table.

In the translating step, the base sequence of the oligonucleotide that is amplified and determined in the determining step is translated based on the relation table, wherein the relation table shows relationships between at least 4^{N} nucleotide N-mer (NH represents a numerical term) and at least the modified nucleotide N-mer, and wherein the 4^{N} nucleotide N-mer (N represents a numerical term) are presented as one-to-one combinations of four kinds of a nucleoside in the relation table.

The translation is preferably carried out for every N base from the 5' terminal side of the modified oligonucleotide sequence made of the modified nucleotide N-mer, of which base sequence has been determined, on the basis of the relation table. It is more preferable to insert a fixed base to define a block periodically. For instance, when the modified nucleotide N-mer is a modified nucleotide dimer, the translation is carried out for every two bases from the 5' terminal side of the modified oligonucleotide sequence, of which base sequence has been determined, made of the modified nucleotide dimer, on the basis of the relation table. Specifically, the translation is carried out on the basis of the following relation table, Table 1.

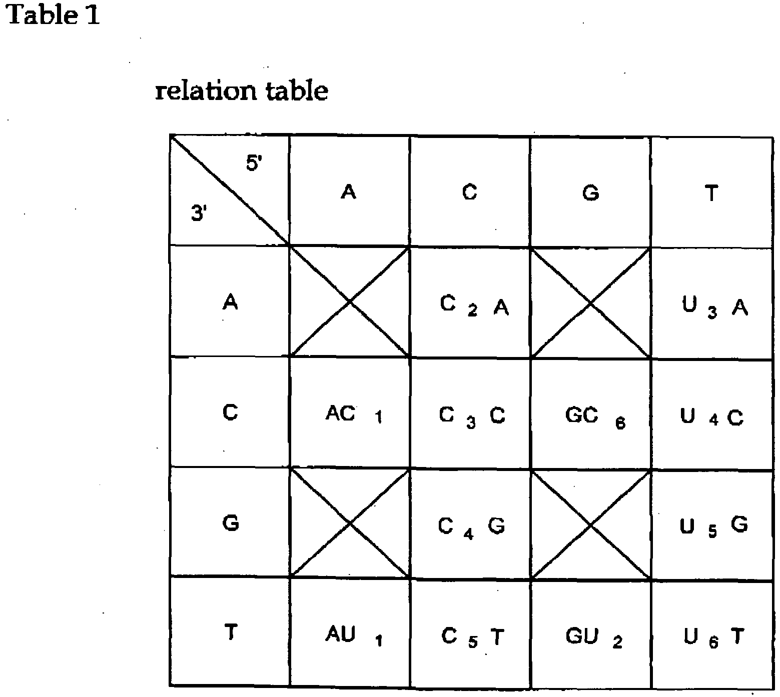

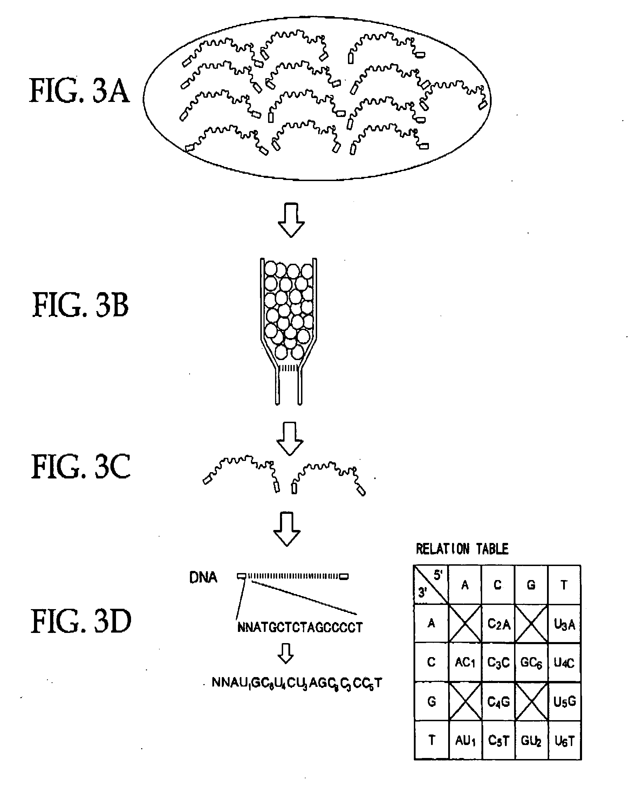

For instance, as shown in FIG. 3, when the determined modified oligonucleotide sequence is "ATGCTCTAGCCCCT," it is confirmed that the sequence is translated into "AU₁GC₆U₄CU₃AGC₆C₃CC₅T" on the basis of the relation table, to thereby determine the sequence of the functional molecule.

When the modified nucleotide N-mer is a modified nucleotide trimer, the translation is carried out for every three bases from the 5' terminal side of the modified oligonucleotide sequence, of which the base sequence has been determined, made of the modified nucleotide trimer on the basis of the relation table. Specifically, the translation is carried out on the basis of the following relation table, Table 2.

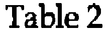

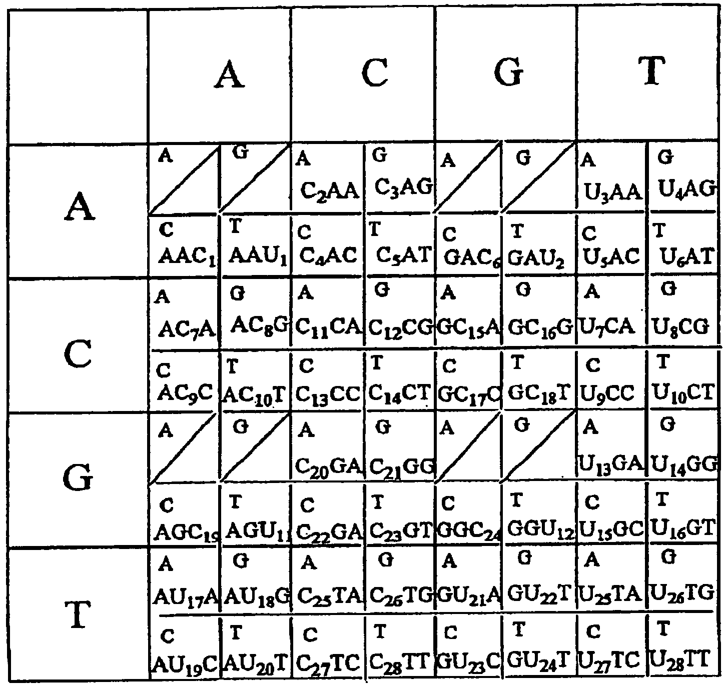

In addition, when the modified nucleotide N-mer is tetramer or larger **(**N **≥** tetra-) nucleotide, the translation is carried out in the same manner, for example, on the basis of the relation table, wherein the relation table shows relationships between at least 4^{N} nucleotide N-mer **(**N **≥** tetra-) and at least the modified nucleotide N-mer, and wherein the 4^{N} nucleotide N-mer (N ≥ tetra-) are presented as one-to-one combinations of four kinds of a nucleoside in the relation table.

The functional molecule according to the present invention obtained by the process for producing a functional molecule according to the present invention has the affinity and specificity comparable to those of the antibody, and can be preferably and widely used in various fields. For instance, the functional molecule according to the present invention, which is found to have affinity for a specific protein and is produced by the process for producing a functional molecule of the present invention, can be suitably used in fields of drugs and drug delivery. Moreover, the functional molecule according to the present invention, which is found to have affinity for a specific DNA sequence and is produced by the process for producing a functional molecule of the present invention, can be suitably used for conquering a disease caused by gene abnormality and discovering a function of a protein translated by the gene. In addition, the functional molecule according to the present invention, which is found to have affinity for a molecule prepared by mixing reaction intermediates and is produced by the process for producing a functional molecule of the present invention, can be suitably used for development of a catalyst that efficiently progresses the reaction.

Examples of the present invention will be described hereinafter, but the present invention is not restricted by these examples.

### (Example 1)

A functional molecule having affinity for a specific protein was produced, i.e. being synthesized and identified, as illustrated in FIG. 3. Specifically, at first, six kinds of deoxycytidine relatives (C₁₋₆) having a functional group at the 5th position of cytosine and six kinds of deoxyuridine relatives (U₁₋₆) having a functional group at the 5th position of uracil were synthesized (produced) in the manner as illustrated in the following formulae.

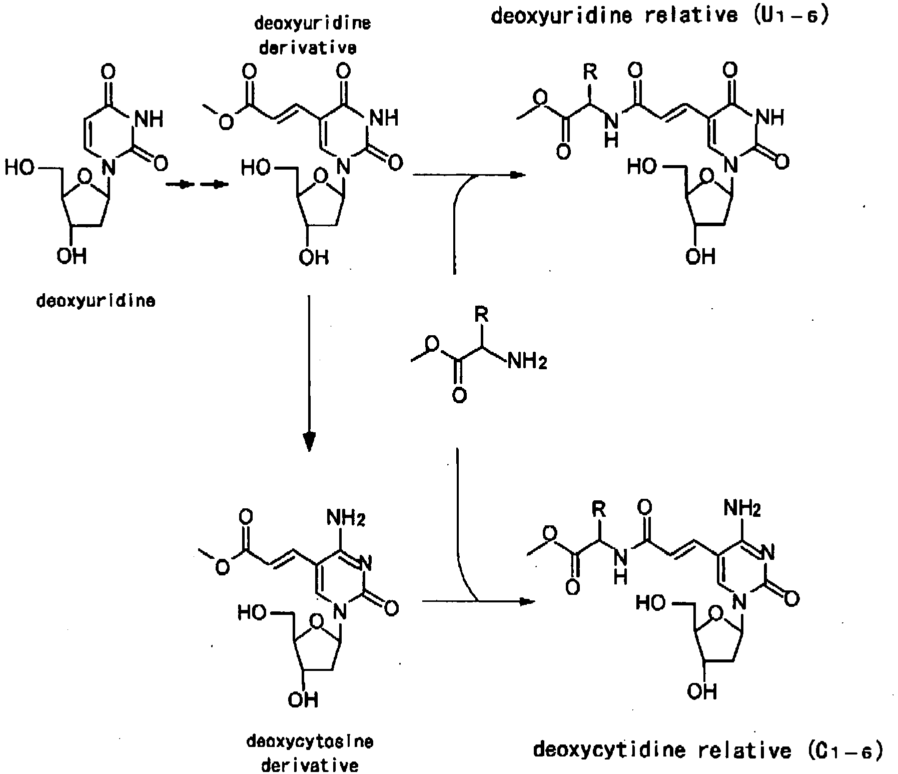

| | | | |
|---|---|---|---|
| R¹ : | -H | R⁷ : | -CH₃ |
| R² : | 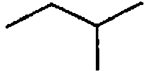 | R⁸ : | 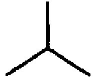 |
| R³: | 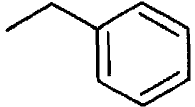 | R⁹ : | 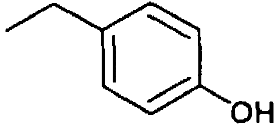 |
| R⁴: | 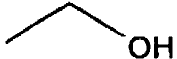 | R¹⁰ : | 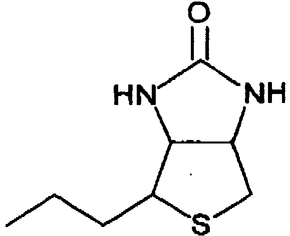 |
| R⁵ : | 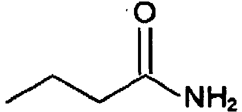 | | |
| R⁶ : | 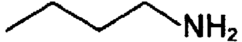 | R¹¹ : | 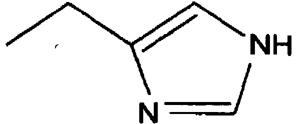 |
| | | R¹² : | 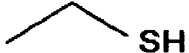 |

Next, each one of twelve kinds of the synthesized (produced) modified nucleoside dimers was related to each one of sixteen patterns which were combinations of four types of nucleotide composing DNA presented in the following Table 1.

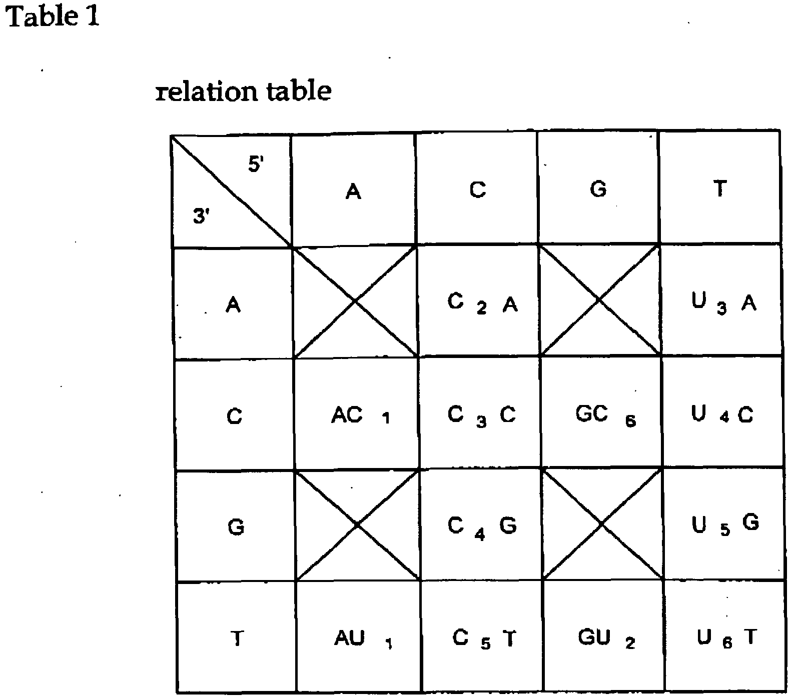

Next, twelve kinds of the modified oligonucleotide amidite (M) represented in the above relation table, Table 1 were chemically synthesized in accordance with the phosphoroamidite method. Specifically, for instance, the modified nucleotide dimer (AU₁) can be synthesized from deoxyadenosine and modified deoxyuridine as illustrated in the following formulae.

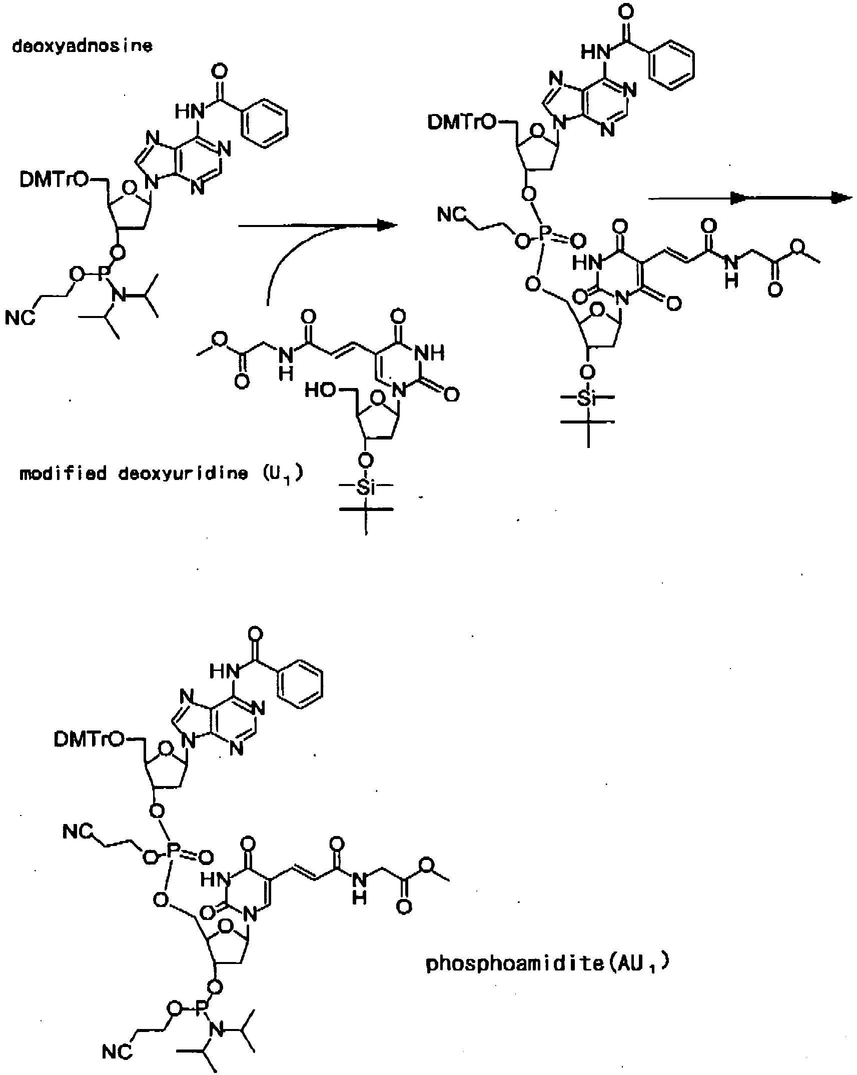

In the above formulae, DMTr represents a dimethoxytrityl group.

Thereafter, a random polymer pool was prepared by means of a DNA synthesizer as shown in FIG. 2. The random polymer pool contained a random oligonucleotide N₂₀-M₂₀-N₂₀ (DNA 80-mer) consisting of a fixed oligonucleotide sequence 20-mer (N₂₀) - a modified oligonucleotide random sequence 20-mer (M₂₀) - a fixed oligonucleotide sequence 20-mer (N₂₀). The above described is the producing step.

Subsequently, the modified oligonucleotide sequence (functional molecule), which was strongly bound to the specific protein, was identified by using this random polymer pool (FIG. 3A). In FIG. 3A, an open square "□" expresses the fixed nucleotide sequence and a wavy line "~" expresses the modified nucleotide random sequence.

First, an affinity column, which was loaded with beads to which human albumin as the target was bound, was prepared and a solution containing the random polymer pool was passed through this column and left under the specific condition. Then, the oligonucleotide containing the modified oligonucleotide sequence showing affinity for the target was adsorbed to the column (FIG. 3B). Subsequently, components other than the adsorbed (remained) modified oligonucleotide sequence were removed by sufficiently washing with a buffer solution, and the solution containing human albumin was passed through the column so as to select and collect the oligonucleotide containing the modified oligonucleotide random sequence (FIG. 3C). The described above is the selecting step.

This selected modified oligonucleotide sequence was amplified by PCR, followed by cloning to thereby determine the base sequence of the DNA. As a result, it was found out that a part of the base sequence of the determined modified oligonucleotide was "ATGCTCTAGCCCCT" (FIG. 3D). The described above is the determining step.

This base sequence was translated on the basis of the relation table above, and the translation thereof was resulted as AU₁GC₆U₄CU₃AGC₆C₃CC₅T". In this way, the structure of the functional molecule was identified (FIG. 3D). The described above is the translating step.

It was found that the resulting functional molecule was specifically bound to human albumin so as to function similarly to the antibody.

### (Example 2)

As a random polymer pool containing the modified oligonucleotide sequence, a 5'-³²P radiolabeled standard (that prepared by previously labeling a nucleotide monomer containing a modified base with a radioactive element) of the DNA random pool (total amount 20 OD) [nucleotide fixed sequence 20-mer DNA (N₂₀)] synthesized in Example 1 was prepared. The step described above is the step of producing the modified oligonucleotide sequence.

Meanwhile, a cytoplasm fraction was purified from a rat liver tissue in the conventional manner. A protein mixture contained in this fraction was subjected to linear isoelectric focusing (pH 4 to 7) in the conventional manner, followed by SDS-PAGE as two-dimensional electrophoresis. After carrying out the electrophoresis, the protein contained in the protein mixture was blotted onto a nitrocellulose membrane by the western blotting method.

The nitrocellulose membrane, on which the protein mixture was immobilized, was soaked, and shaken for 1 hour, in a diluted solution of N₂₀M₂₀N₂₀ (DNA 80-mer) that was the random polymer pool containing the radiolabeled modified oligonucleotide sequence. Thereafter, this nitrocellulose membrane was washed with the buffer solution so as to remove nonspecific remains, and then a position (the position to which the radiolabeled modified oligonucleotide was strongly bound), where strong radioactivity was observed, of the nitrocellulose membrane was identified by using an X-ray film.

Thirty positions, where strong radioactivity was observed, of the nitrocellulose membrane were cut out, and the cut pieces were applied with a voltage in an ionic solution (3M Na acetate, pH 4.8, 0.1% bromophenol blue solution) so as to collect the modified oligonucleotide in accordance with electroelution. After the observation of radioactivity of each solution, the DNA was amplified by PCR using a complementary chain of a fixed sequence as the primer. PCR products were each subjected to acrylamide gel electrophoresis. As a result, distinct bands of twenty-eight kinds of the modified oligonucleotide sequences were detected. The step described above is the selecting step.

The obtained twenty-eight kinds of the modified oligonucleotide sequences were each subjected to cloning by using plasmid vectors so as to determine the DNA sequences. The step described above is the determining step. After this step, the sequence of the modified DNA was determined by the same manner as described in Example 1 to synthesize the modified DNA (the functional molecule) by means of an automated DNA synthesizer. The step described above is the translating step.

It was observed that the obtained modified DNA could specifically recognize the protein isolated by two-dimensional electrophoresis. Further, the dissociation constant of an available protein was measured by applying a surface plasmon resonance method. As a result, it was found out that the dissociation constant was 1.0 × 10⁻⁷ to 1.0 × 10⁻⁹ and the protein showed the affinity.

The same effect was obtained in the case where native electrophoresis instead of SDS-PAGE, as the second dimensional electrophoresis was applied to isolate the protein while maintaining its molecular structure.

In Example 2, the labeling material usable in a radiolabeled random polymer pool includes, in addition to the radioisotope, those selected from labeling materials such as enzymes, fluorescent materials, and chemiluminescence materials. In addition, transferred proteins on a support such as a blotted nitrocellulose membrane for a transferring operation can be visualized by staining or spectroscopy to extract a DNA sequence from a region having abundant transferred proteins for cloning and sequencing.

### Industrial Applicability

According to the present invention, various problems in the prior art can be solved, and there provides a functional molecule that has affinity and specificity for a target, which are comparable to the affinity and specificity of an antibody against an antigen, that functions similarly to the antibody sensu stricto, that is capable of recognizing a wide range of targets, that shows high affinity for various kinds of targets (targeted material and detection target), that is easily amplified and replicated, that is suitable for use in various fields such as medical drugs, drug delivery, biosensors, regulation of gene expression level, conquest of diseases caused by an abnormal gene, and analyzing the function of the protein translated by a gene, development of reaction catalysts, and that is particularly suitable for an analysis of a protein.

The present invention provides a process for producing a functional molecule, which can efficiently and collectively produce functional molecule(s) without repeating complicated operations of amplification and purification.

## Claims

1. A process for producing a functional molecule having affinity for a target, comprising the steps of:
a) choosing a numerical term N, which is an integer and 2 or larger;
b) providing between 5 and 4^{N} varieties of modified nucleotide N-mers, each comprising a modified nucleoside prepared by introducing a substituent into a nucleoside composing a nucleic acid, and wherein each modified nucleotide N-mer is represented in a relation table which shows the correspondence of each modified nucleotide N-mer to one of the 4^{N} possible unmodified nucleotide N-mer base sequences;
c) randomly polymerizing the modified nucleotide N-mers so as to produce modified oligonucleotide sequences;
d) producing a random polymer pool comprising the modified oligonucleotide sequences;
e) contacting a target with the random polymer pool;
f) selecting a modified oligonucleotide sequence having affinity for the target from the random polymer pool;
g) PCR amplifying the selected modified oligonucleotide, and determining the unmodified base sequence thereof;
h) translating every N bases from the 5' terminal side of the unmodified oligonucleotide sequence into modified nucleotide N-mer sequences on the basis of the relation table;
i) combining the translated modified N-mer sequences into a modified oligonucleotide sequence, which corresponds to the selected modified oligonucleotide of step f);
j) producing the modified oligonucleotide having the sequence obtained in step i).

2. The process for producing a functional molecule according to claim 1, wherein N is 2 or 3.

3. The process for producing a functional molecule according to claim 1 or claim 2, wherein the nucleic acid is either DNA or RNA.

4. The process for producing a functional molecule according to any one of claims 1 to 3, wherein the substituent is introduced at a 5th position of a pyrimidine base of the nucleoside.

5. The process for producing a functional molecule according to any of claim 1 to claim 3, wherein the substituent is selected from groups represented by the following structural formula (I): 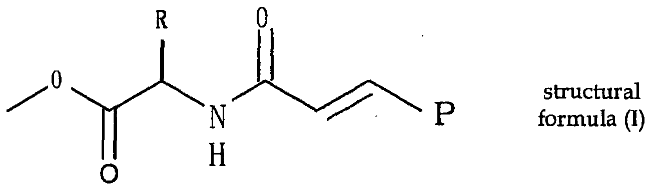 where, in the formula (I), R represents any one of groups selected from natural or unnatural amino acids, metal complexes, fluorescent dyes, oxidation-reduction coloring agents, spin label compounds, hydrogen atom, C1-10 alkyl groups, and groups selected from the following formulae (1) to (10); and P represents a pyrimidine base.

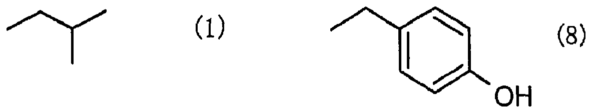

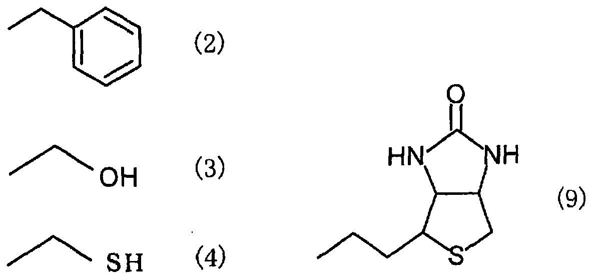

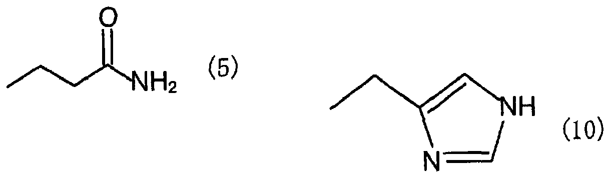

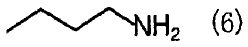

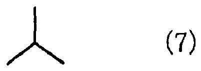

6. The process for producing a functional molecule according to any of claim 1 to claim 5, wherein the modified nucleotide N-mers comprise the modified oligonucleotide amidite.

7. The process for producing a functional molecule according to any of claim 1 to claim 6, wherein the modified oligonucleotide sequences are synthesized by using a DNA synthesizer.

8. The process for producing a functional molecule according to any of claim 1 to claim 6, wherein each of the modified oligonucleotide sequences is synthesized by annealing nucleotide monomers to a nucleotide random sequence, and joining the nucleotide monomers each other by using at least one of a DNA ligase and an RNA ligase.

9. The process for producing a functional molecule according to any of claim 1 to claim 8, wherein each of the modified oligonucleotide sequences has fixed oligonucleotide sequences at both terminals thereof.

10. The process for producing a functional molecule according to claim 1, wherein the selecting step is carried out by affinity chromatography employing a column in which beads bound with a target are loaded.

11. The process for producing a functional molecule according to claim 1, wherein the selecting step is carried out by dissociating the modified oligonucleotide sequence from the target while monitoring dissociation constants of the target and the modified oligonucleotide sequence.

12. The process for producing a functional molecule according to claim 1, wherein the selecting step is carried out in a manner that 2 or more targets are immobilized on a matrix having one to three dimensional spatial arrangement, the random polymer pool containing the modified oligonucleotide sequence is allowed to interact with the matrix on which the target has been immobilized, and then the modified oligonucleotide sequence attached to the target is purified.

13. The process for producing a functional molecule according to claim 12, wherein the immobilization of the target on the matrix is carried out by any one method selected from a western blotting method, a dot blotting method, and a slot blotting method.

14. The process for producing a functional molecule according to one of claim 12 and claim 13, wherein the target immobilized on the matrix is labeled with at least one labeling material selected from radioisotopes, chemiluminescence materials, fluorescent materials, enzymes, antibodies, ligands and receptors.

15. The process for producing a functional molecule according to any one of claim 11 to claim 14, wherein the modified oligonucleotide sequence contained in the random polymer pool is labeled with at least one labeling material selected from radioisotopes, chemiluminescence materials, fluorescent materials, enzymes, antibodies, ligands, and receptors.

16. The process for producing a functional molecule according to any one of claim 11 to claim 15, wherein the selecting step is carried out in a manner that the matrix on which the modified oligonucleotide sequence is immobilized is cut to thereby yield a cut piece, and then PCR is directly conducted on the cut piece, or the modified oligonucleotide sequence is collected from the cut piece.

17. The process for producing a functional molecule according to claim 16, wherein the collection of the modified oligonucleotide sequence from the cut piece is carried out by one of an electroelution method applying a voltage and using an ionic solution, and a method using a hot water.

18. The process for producing a functional molecule according to any one of claim 1 to claim 17, wherein the target is at least one selected from proteins, lipoproteins, sugar proteins, polypeptides, lipids, polysaccharides, lipopolysaccharides, nucleic acids, environmental hormones, drugs, and a complex of these substances.

19. The process for producing a functional molecule according to claim 1, wherein the PCR amplification is carried out in accordance with any one method selected from PCR and RT-PCR , and the base sequence is determined by cloning.

## Patentansprüche

1. Prozess zum Herstellen eines funktionellen Moleküls, das Affinität zu einem Target hat, mit den Schritten:
a) Wählen eines Zahlenterms N, der eine ganze Zahl größer gleich 2 ist;
b) Vorsehen zwischen 5 und 4^{N} Varianten von modifizierten Nucleotid-N-meren, die jeweils ein modifiziertes Nucleosid enthalten, das vorbereitet wird durch Einführen eines Substituenten in ein Nucleosid, das eine Nucleinsäure bildet, und bei dem jedes modifizierte Nucleotid-N-mer in einer Beziehungstabelle dargestellt wird, welche die Entsprechung jedes modifizierten Nucleotid-N-mers zu einer der 4^{N} möglichen unmodifizierten Nucleotid-N-mer-Basensequenzen zeigt;
c) Zufallspolymerisation der modifizierten Nucleotid-N-mere, um modifizierte Oligonucleotidsequenzen zu produzieren;
d) Produzieren eines Pools von Zufallspolymeren, der die modifizierten Oligonucleotidsequenzen umfasst;
e) Kontaktieren eines Targets mit dem Pool von Zufallspolymeren;
f) Auswählen einer modifizierten Oligonucleotidsequenz, die Affinität zu dem Target hat, aus dem Pool von Zufallspolymeren;
g) PCR-Amplifikation des ausgewählten modifizierten Oligonucleotids und Bestimmen der unmodifizierten Basensequenz desselben;
h) Übersetzen aller N Basen vom 5'-Ende der unmodifizierten Oligonucleotidsequenz in modifizierte Nucleotid-N-mer-Sequenzen auf der Basis der Beziehungstabelle;
i) Kombinieren der übersetzten modifizierten N-mer-Sequenzen zu einer modifizierten Oligonucleotidsequenz, die dem ausgewählten modifizierten Oligonucleotid von Schritt f) entspricht;
j) Produzieren des modifizierten Oligonucleotids, das die bei Schritt i) erhaltene Sequenz hat.

2. Prozess zum Herstellen eines funktionellen Moleküls nach Anspruch 1, bei dem N 2 oder 3 ist.

3. Prozess zum Herstellen eines funktionellen Moleküls nach Anspruch 1 oder 2, bei dem die Nucleinsäure entweder eine DNA oder eine RNA ist.

4. Prozess zum Herstellen eines funktionellen Moleküls nach einem der Ansprüche 1 bis 3, bei dem der Substituent an einer 5. Position einer Pyrimidinbase des Nucleosids eingeführt wird.

5. Prozess zum Herstellen eines funktionellen Moleküls nach einem der Ansprüche 1 bis 3, bei dem der Substituent ausgewählt wird aus Gruppen, die durch die folgende Strukturformel (I) dargestellt werden: 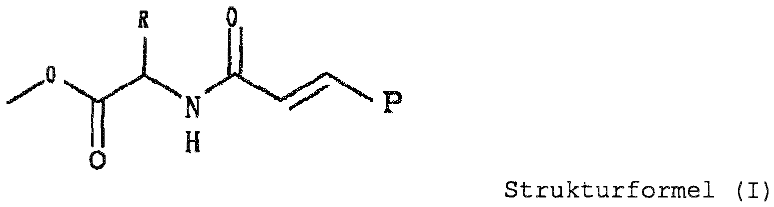 wobei R in der Formel (I) eine der Gruppen darstellt, die ausgewählt werden aus natürlichen oder unnatürlichen Aminosäuren, Metallkomplexen, Fluoreszenzfarbstoffen, Oxidationsreduktionsfärbemitteln, Spinmarkierungsverbindungen, einem Wasserstoffatom, C1-10-Alkylgruppen und Gruppen, die ausgewählt werden aus den folgenden Formeln (1) bis (10), und P eine Pyrimidinbase darstellt.

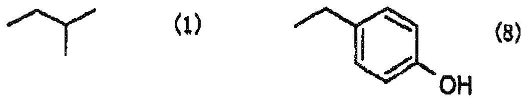

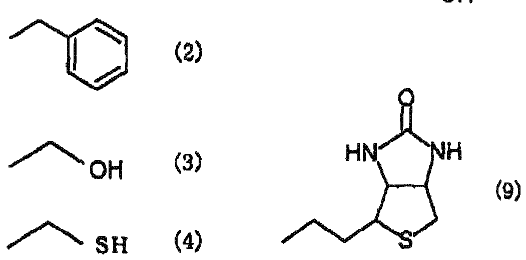

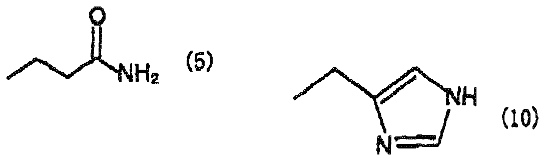

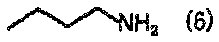

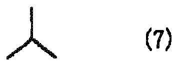

6. Prozess zum Herstellen eines funktionellen Moleküls nach einem der Ansprüche 1 bis 5, bei dem die modifizierten Nucleotid-N-mere das modifizierte Oligonucleotid Amidit umfassen.

7. Prozess zum Herstellen eines funktionellen Moleküls nach einem der Ansprüche 1 bis 6, bei dem die modifizierten Oligonucleotidsequenzen unter Verwendung eines DNA-Synthesizers synthetisiert werden.

8. Prozess zum Herstellen eines funktionellen Moleküls nach einem der Ansprüche 1 bis 6, bei dem jede der modifizierten Oligonucleotidsequenzen synthetisiert wird, indem Nucleotidmonomere für eine Nucleotidzufallssequenz annealt werden und die Nucleotidmonomere unter Verwendung von wenigstens einer von einer DNA-Ligase und einer RNA-Ligase miteinander verbunden werden.

9. Prozess zum Herstellen eines funktionellen Moleküls nach einem der Ansprüche 1 bis 8, bei dem jede der modifizierten Oligonucleotidsequenzen konstante Oligonucleotidsequenzen an ihren beiden Enden hat.

10. Prozess zum Herstellen eines funktionellen Moleküls nach Anspruch 1, bei dem der Auswählschritt durch Affinitätschromatographie unter Verwendung einer Säule ausgeführt wird, in die Perlen gefüllt sind, die an ein Target gebunden sind.

11. Prozess zum Herstellen eines funktionellen Moleküls nach Anspruch 1, bei dem der Auswählschritt durch Dissoziieren der modifizierten Oligonucleotidsequenz von dem Target unter Überwachung von Dissoziationskonstanten des Targets und der modifizierten Oligonucleotidsequenz ausgeführt wird.

12. Prozess zum Herstellen eines funktionellen Moleküls nach Anspruch 1, bei dem der Auswählschritt in einer Weise ausgeführt wird, dass 2 oder mehr Targets auf einer Matrix mit ein- bis dreidimensionaler räumlicher Anordnung immobilisiert werden, der Pool von Zufallspolymeren, der die modifizierte Oligonucleotidsequenz enthält, mit der Matrix, auf der das Target immobilisiert worden ist, interagieren kann und dann die modifizierte Oligonucleotidsequenz, die sich an dem Target angelagert hat, purifiziert wird.

13. Prozess zum Herstellen eines funktionellen Moleküls nach Anspruch 12, bei dem die Immobilisierung des Targets auf der Matrix durch eine Methode ausgeführt wird, die ausgewählt wird aus einer Western-Blot-Methode, einer Dot-Blot-Methode und einer Slot-Blot-Methode.

14. Prozess zum Herstellen eines funktionellen Moleküls nach einem der Ansprüche 12 und 13, bei dem das auf der Matrix immobilisierte Target mit wenigstens einem Markierungsmaterial markiert wird, das ausgewählt wird aus Radioisotopen, Chemilumineszenzmaterialien, Fluoreszenzmaterialien, Enzymen, Antikörpern, Liganden und Rezeptoren.

15. Prozess zum Herstellen eines funktionellen Moleküls nach einem der Ansprüche 11 bis 14, bei dem die modifizierte Oligonucleotidsequenz, die in dem Pool von Zufallspolymeren enthalten ist, mit wenigstens einem Markierungsmaterial markiert wird, das ausgewählt wird aus Radioisotopen, Chemilumineszenzmaterialien, Fluoreszenzmaterialien, Enzymen, Antikörpern, Liganden und Rezeptoren.

16. Prozess zum Herstellen eines funktionellen Moleküls nach einem der Ansprüche 11 bis 15, bei dem der Auswählschritt in einer Weise ausgeführt wird, dass die Matrix, auf der die modifizierte Oligonucleotidsequenz immobilisiert ist, zerschnitten wird, um dadurch einen Abschnitt zu erhalten, und dann die PCR direkt auf dem Abschnitt durchgeführt wird oder die modifizierte Oligonucleotidsequenz von dem Abschnitt entnommen wird.

17. Prozess zum Herstellen eines funktionellen Moleküls nach Anspruch 16, bei dem die Entnahme der modifizierten Oligonucleotidsequenz von dem Abschnitt durch eine Elektroelutionsmethode, bei der eine Spannung angewendet wird und eine Ionenlösung verwendet wird, oder eine Methode unter Verwendung von heißem Wasser ausgeführt wird.

18. Prozess zum Herstellen eines funktionellen Moleküls nach einem der Ansprüche 1 bis 17, bei dem das Target wenigstens eines ist, das ausgewählt wird aus Proteinen, Lipoproteinen, Zuckerproteinen, Polypeptiden, Lipiden, Polysacchariden, Lipopolysacchariden, Nucleinsäuren, Umwelthormonen, Drogen und einem Komplex dieser Substanzen.

19. Prozess zum Herstellen eines funktionellen Moleküls nach Anspruch 1, bei dem die PCR-Amplifikation gemäß einer Methode ausgeführt wird, die ausgewählt wird aus einer PCR und RT-PCR, und die Basensequenz durch Klonen bestimmt wird.

## Revendications

1. Procédé pour produire une molécule fonctionnelle ayant une affinité pour une cible, comprenant les étapes de :
a) choisir un terme numérique N qui est un entier et 2 ou plus ;
b) fournir entre 5 et 4^{N} variétés de N-mères nucléotidiques modifiés, comprenant chacun un nucléoside modifié préparé par introduction d'un substituant dans un nucléoside composant un acide nucléique, et où chaque N-mère nucléotidique modifié est représenté dans une table de relations qui montre la correspondance de chaque N-mère nucléotidique modifié à l'une des 4^{N} séquences de bases de N-mères nucléotidiques non modifiées possibles ;
c) polymériser par polymérisation statistique les N-mères nucléotidiques modifiés de manière à produire des séquences oligonucléotidiques modifiées ;
d) produire un groupe de polymères statistiques comprenant les séquences oligonucléotidiques modifiées ;
e) mettre en contact une cible avec le groupe de polymères statistiques ;
f) sélectionner une séquence oligonucléotidique modifiée ayant une affinité pour la cible parmi le groupe de polymères statistiques ;
g) amplifier par PCR l'oligonucléotide modifié sélectionné, et déterminer sa séquence de bases non modifiée ;
h) traduire toutes les N bases depuis le côté 5' terminal de la séquence oligonucléotidique non modifiée en séquences de N-mères nucléotidiques modifiées sur la base de la table de relations ;
i) combiner les séquences de N-mères modifiées traduites en une séquence oligonucléotidique modifiée, qui correspond à l'oligonucléotide modifié sélectionné de l'étape f) ;
j) produire l'oligonucléotide modifié ayant la séquence obtenue dans l'étape i).

2. Procédé pour produire une molécule fonctionnelle selon la revendication 1, où N est 2 ou 3.

3. Procédé pour produire une molécule fonctionnelle selon la revendication 1 ou la revendication 2, où l'acide nucléique est un ADN ou un ARN.

4. Procédé pour produire une molécule fonctionnelle selon l'une quelconque des revendications 1 à 3, où le substituant est introduit à une cinquième position d'une base pyrimidique du nucléoside.

5. Procédé pour produire une molécule fonctionnelle selon l'une quelconque de la revendication 1 à la revendication 3, où le substituant est choisi parmi les groupes représentés par la formule structurale (I) suivante : 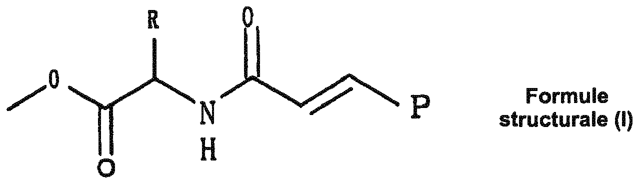 où, dans la formule (I), R représente l'un quelconque de groupes choisis parmi les aminoacides naturels ou non naturels, les complexes métalliques, les colorants fluorescents, les agents colorants d'oxydation-réduction, les composés marqueurs de spin, un atome d'hydrogène, les groupes C1-10 alkyle et les groupes choisis parmi les formules (1) à (10) suivantes ; et P représente une base pyrimidique.

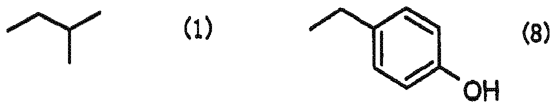

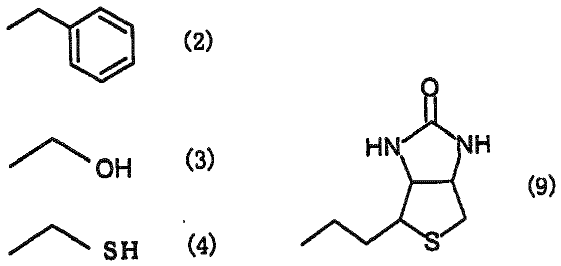

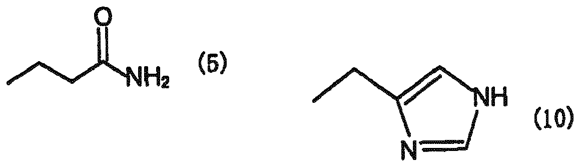

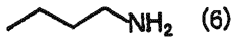

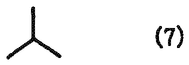

6. Procédé pour produire une molécule fonctionnelle selon l'une quelconque de la revendication 1 à la revendication 5, où les N-mères nucléotidiques modifiés comprennent l'oligonucléotide amidite modifié.

7. Procédé pour produire une molécule fonctionnelle selon l'une quelconque de la revendication 1 à la revendication 6, où les séquences oligonucléotidiques modifiées sont synthétisées au moyen d'un synthétiseur d'ADN.

8. Procédé pour produire une molécule fonctionnelle selon l'une quelconque de la revendication 1 à la revendication 6, où chacune des séquences oligonucléotidiques modifiées est synthétisée par annelage de monomères nucléotidiques à une séquence statistique nucléotidique, et jonction des monomères nucléotidiques entre eux en utilisant au moins l'une d'une ADN ligase et d'une ARN ligase.

9. Procédé pour produire une molécule fonctionnelle selon l'une quelconque de la revendication 1 à la revendication 8, où chacune des séquences oligonucléotidiques modifiées a des séquences oligonucléotidiques fixées à ses deux extrémités.

10. Procédé pour produire une molécule fonctionnelle selon la revendication 1, où l'étape de sélection est conduite par chromatographie d'affinité employant une colonne dans laquelle des billes liées à une cible sont introduites.

11. Procédé pour produire une molécule fonctionnelle selon la revendication 1, où l'étape de sélection est conduite par dissociation de la séquence oligonucléotidique modifiée d'avec la cible tout en suivant les constantes de dissociation de la cible et de la séquence oligonucléotidique modifiée.

12. Procédé pour produire une molécule fonctionnelle selon la revendication 1, où l'étape de sélection est conduite d'une manière selon laquelle deux ou plusieurs cibles sont immobilisées sur une matrice ayant un à trois agencements spatiaux dimensionnels, le groupe de polymères statistiques contenant la séquence oligonucléotidique modifiée est amenée à interagir avec la matrice sur laquelle la cible a été immobilisée, puis la séquence oligonucléotidique modifiée fixée à la cible est purifiée.

13. Procédé pour produire une molécule fonctionnelle selon la revendication 12, où l'immobilisation de la cible sur la matrice est conduite par un procédé quelconque choisi parmi un procédé de transfert de western, un procédé de transfert en taches, et un procédé de transfert en fentes.

14. Procédé pour produire une molécule fonctionnelle selon l'une de la revendication 12 et de la revendication 13, où la cible immobilisée sur la matrice est marquée avec au moins un matériau marqueur choisi parmi les radio-isotopes, les matériaux chimiluminescents, les matériaux fluorescents, les enzymes, les anticorps, les ligands et les récepteurs.

15. Procédé pour produire une molécule fonctionnelle selon l'une quelconque de la revendication 11 à la revendication 14, où la séquence oligonucléotidique modifiée contenue dans le groupe de polymères statistiques est marquée avec au moins un matériau marqueur choisi parmi les radio-isotopes, les matériaux chimiluminescents, les matériaux fluorescents, les enzymes, les anticorps, les ligands et les récepteurs.

16. Procédé pour produire une molécule fonctionnelle selon l'une quelconque de la revendication 11 à la revendication 15, où l'étape de sélection est conduite d'une manière telle que la matrice sur laquelle la séquence oligonucléotidique modifiée est immobilisée est coupée pour produire ainsi un fragment coupé, puis une PCR est conduite directement sur le fragment coupé, ou bien la séquence oligonucléotidique modifiée est récupérée à partir du fragment coupé.

17. Procédé pour produire une molécule fonctionnelle selon la revendication 16, où la récupération de la séquence oligonucléotidique modifiée à partir du fragment coupé est réalisée par l'un d'un procédé d'électro-élution appliquant une tension et utilisant une solution ionique et d'un procédé utilisant de l'eau chaude.

18. Procédé pour produire une molécule fonctionnelle selon l'une quelconque de la revendication 1 à la revendication 17, où la cible est au moins une choisie parmi les protéines, les lipoprotéines, les protéines glucidiques, les polypeptides, les lipides, les polysaccharides, les lipopolysaccharides, les acides nucléiques, les hormones environnementales, les médicaments et un complexe de ces substances.

19. Procédé pour produire une molécule fonctionnelle selon la revendication 1, où l'amplification par PCR est conduite selon un procédé quelconque choisi parmi la PCR et la RT-PCR, et la séquence de bases est déterminée par clonage.
